# EUROPEAN PATENT SPECIFICATION

(11) **EP 2 226 066 B1**
(45) Date of publication and mention of the grant of the patent: **04.05.2016**
(21) Application number: 08868137.4
(22) Date of filing: 25.12.2008
(51) Int. Cl.: A61K 8/49, A61K 8/97, A61K 45/00, A61K 47/34, A61P 17/00, A61Q 19/00, A61K 8/86, A61Q 17/04, A61K 31/575, A61K 47/28, A61K 9/00

(54) **WATER-CONTAINING COMPOSITION COMPRISING A SOLUBILISED TRIAZINE**
WASSERHALTIGE ZUSAMMENSETZUNG ENTHALTEND EIN SOLUBILISIERTES TRIAZIN
COMPOSITION CONTENANT DE L'EAU ET UNE TRIAZINE SOLUBILISÉE

(30) Priority: 27.12.2007 JP 2007336112
(43) Date of publication of application: 08.09.2010
(73) Proprietor: Shiseido Company, Ltd., Chuo-ku Tokyo 104-8010 (JP)
(72) Inventor: TESHIGAWARA, Takashi, Yokohama-shi Kanagawa 224-8558 (JP); MIYAHARA, Reiji, Yokohama-shi Kanagawa 224-8558 (JP); OKA, Takashi, Yokohama-shi Kanagawa 224-8558 (JP)
(74) Representative: Schön, Christoph
(86) International application number: PCT/JP2008/073603
(87) International publication number: WO 2009/084588

(56) References cited:
- EP-A1- 2 305 207
- WO-A1-03/039511
- WO-A1-2006/016549
- WO-A1-2009/016989
- JP-A- 54 129 114
- JP-A- 55 098 109
- JP-A- 2003 040 740
- JP-A- 2006 111 620
- JP-A- 2008 031 074
- JP-T- 2005 511 586

## Description

### Technical Field

The present invention relates to a water-containing composition that can be employed as a composition for external use (hereinafter may be referred to as an "external-use composition") in the form of cosmetic composition or external-use agent.

### Background Art

Among many substances that are difficult to dissolve in water (hereinafter such a substance may be referred to as a "low water-soluble substance"), quite a lot of substances are useful in the field of external-use compositions for the skin including external-use compositions for the scalp or hair (e.g., cosmetic compositions or external-use agents).
Various techniques have been provided for reliably incorporating such a low water-soluble substance into an external-use composition. Typical examples of such a technique include emulsification and solubilization. Dispersion in water or solubilization may be employed as a technique which realizes spreading of a low water-soluble substance on the skin with favorable refreshing sensation.
In particular, solubilization is a technique essential for using a low water-soluble substance in the form of, for example,
lotion. In general, a low water-soluble substance is solubilized by use of a surfactant such as polyoxyethylene alkyl ether
or polyoxyethylene hydrogenated castor oil. However, when a low water-soluble substance having a high molecular weight (in particular, a low water-soluble substance having a bulky structure) is used, much difficulty is encountered in solubilizing a sufficient amount of the low water-soluble substance by use of such a surfactant.
Patent Document 1: Japanese Patent Application Laid-Open (kokai) No. 2005-511586
Disclosure of the Invention
Problems to be Solved by the Invention

Patent Document 1 discloses that ethoxylated phytosterol or phytostanol is used as means for dissolving a low water-soluble pharmaceutical drug. Ethoxylated phytosterol is used as a solubilizing agent, but, due to its poor ability to solubilize the drug, the agent poses a problem in that it requires a large amount of a surfactant for solubilizing the drug.

JP 54 129114 A discloses solubilized compositions comprising a fat-soluble vitamin B6 ester (e.g. pyridoxine dicaprylate, pyridoxine dilaurate or pyridoxine dipalmitate) together with (A) a phytostanol alkylene oxide adduct (e.g. phytostanol ethylene oxide adduct (ethylene oxide content: 10-50 (20-40) mol %) or phytostanol ethylene oxide propylene oxide adduct (ethylene oxide content: 10-40 (15-30) mol%; propylene oxide content: 4-20 (6-16) mol %)) and (B) a sorbitan fatty acid ester(e.g. sorbitan monooleate or sorbitan sesquioleate).
In view of the foregoing, an object of the present invention is to provide means for enhancing ability to solubilize a low water-soluble substance or to disperse the substance in water.

### Means for solving the Problems

In order to achieve the aforementioned object, the present inventors have conducted studies on an ingredient which exhibits excellent ability to solubilize a low water-soluble substance or to disperse the substance in water, and have found that polyoxyethylene-polyoxypropylene-added phytosterol or phytostanol exhibits very excellent ability to solubilize a low water-soluble substance or to disperse the substance in water. The present invention has been accomplished on the basis of this finding.

Accordingly, the present invention provides a water-containing composition comprising water; at least one polyoxyethylene-polyoxypropylene-added phytosterol or phytostanol represented by the following formula (1): 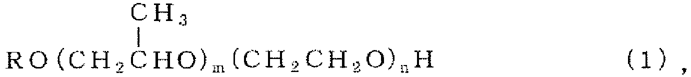 wherein R represents a phytosterol residue or a phytostanol residue, m is a number from 5 to 50, and n is a number from 10 to 50, the ratio of m to n being 1:5 to 5:1, the amount of the polyoxyethylene-polyoxypropylene-added phytosterol or phytostanol (1) being 10 mass% or less on the basis of the entirety of the composition; and
at least one low water-soluble substance which has, in the chemical structure thereof, two or more 6-membered rings, the low water-soluble substance being a triazine UV-absorbing agent,
wherein the low water-soluble substance is in a solubilization state (hereinafter the water-containing composition may be referred to as "the water-containing composition of the present invention"). As used herein, the term "water-containing composition" refers to a composition containing water. As used herein, the term "dissolved state" refers to the case where, when a low water-soluble substance contained in a composition is visually observed under no influence of other ingredients, the substance is found to be uniformly present therein in a transparent or semi-transparent state. The "transparent or semi-transparent state" may be, for example, a state where a low water-soluble substance is thermodynamically stable (i.e., "solubilization state"), or a state where a low water-soluble substance is dispersed in the form of fine particles in the aqueous phase (i.e., "water dispersion state").

Typically, the water-containing composition of the present invention may be employed as an external-use composition that can be applied to the skin, scalp, or hair, or may be employed as a base of the composition. Specific product forms of such an external-use composition include cosmetic compositions and external-use agents. Both "cosmetic composition" and "external-use agent" are in a form that can be applied to the skin, scalp, or hair, and the concepts of these words mutually overlap (in particular, the term "cosmetic composition" encompasses an external-use agent). The case where the external-use composition is employed as an external-use agent rather than a cosmetic composition corresponds to a case where the external-use agent is not a "cosmetic product" for the primary purpose of beauty, but a "quasi-drug" or "drug" for the primary purpose of healthcare.

### Effects of the Invention

According to the present invention, there is provided very excellent means for solubilizing a low water-soluble substance.

### Best Modes for Carrying Out the Invention

As described above,the water-containing composition of the present invention contains compound(1) together with water and a low water-soluble substance. As described hereinbelow, compound (1) contained in the water-containing composition of the present invention may be only one compound represented by the following formula (1), or a mixture of two or more different compounds represented by the following formula (1): 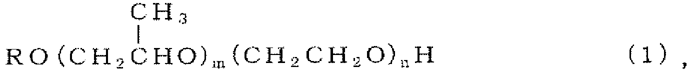 [wherein R represents a phytosterol residue or a phytostanol residue; m is a number from 5 to 50; and n is a number from 10 to 50].

No particular limitation is imposed on the phytosterol, which is a compound that provides a phytosterol residue represented by R in compound (1), and is a plant-derived sterol (F. D. Gunstone and B. G. Herslof, A Lipid Glossary, The Oily Press, Air, 1992). Examples of the phytosterol residue represented by R include a sitosterol residue, a campesterol residue, a stigmasterol residue, a brassicasterol residue, an avenasterol residue, and an ergosterol residue. As described above, compound (1) contained in the water-containing composition of the present invention may be a mixture of two or more compounds having different phytosterol residues represented by R.

Similarly, no particular limitation is imposed on the phytostanol, which is a compound that provides a phytostanol residue represented by R in compound (1), and is obtained through hydrogenation (or saturation) of the corresponding phytosterol. Examples of the phytostanol residue represented by R include sitostanol, campestanol, stigmastanol, brassicastanol, avenastanol, and ergostanol. As described above, compound (1) contained in the water-containing composition of the present invention may be a mixture of two or more compounds having different phytostanol residues represented by R.

Compound (1) contained in the water-containing composition of the present invention may be a mixture of at least one compound having a phytosterol residue represented by R and at least one compound having a phytostanol residue represented by R.

In compound (1), m (i.e., the number representing the polyoxypropylene chain length) is 5 to 50, and n (i.e., the number representing the polyoxyethylene chain length) is 10 to 50.

The ratio of m to n is 1: 5 to 5: 1, from the viewpoints of suppressing stickiness of the water-containing composition of the present invention (i.e., providing a favorable sensation upon use thereof) and securing better stability over time.

Compound (1), which has the aforementioned chemical structure, may be produced through a customary method. For example, compound (1) may be readily produced through the following procedure: propylene oxide is added to phytosterol in the presence of a catalyst (i.e., first-step addition reaction); unreacted matter is removed; ethylene oxide is added to the resultant product (i.e., second-step addition reaction); and unreacted matter is again removed, follower by neutralization, dehydration, deodorization, and filtration.

The amount of compound (1) contained in the water-containing composition of the present invention is 10 mass% or less, preferably 3 mass% or less, on the basis of the entirety of the composition. The amount of compound
(1) greatly depends on the relationship between the amount thereof and that of the low water-soluble substance contained in the composition. The ratio by mass of compound (1) to low water-soluble substance is preferably 1 or more, particularly preferably 3 or more (the upper limit of this ratio is determined on the basis of the upper limit of the amount of compound (1)). A suitable lower limit of the amount of compound (1) may be determined on the basis of this ratio by mass.

The water-containing composition of the present invention must contain at least one low water-soluble substance, which is to be solubilized, said low water-soluble substance having, in the chemical structures thereof, two or more 6-membered rings wherein said low water-soluble substance is a triazine UV-absorbing agent.

As used herein, the term "low water-soluble substance" literally refers to a substance which is insoluble or difficult to dissolve in water. No particular limitation is imposed on the solubility of the low water-soluble substance in a solvent other than water.

The low water-soluble substance is a triazine UV-absorbing agent (e.g., bisresorcinyltriazine, bisethylhexyloxyphenol methoxyphenyltriazine(2,4-bis{[4-(2-ethylhexyloxy)-2-hydroxy]phenyl}-6-(4-methoxyphenyl) 1,3,5-triazine), and octyl triazone (2,4, 6- tris[4-(2-ethylhexyloxycarbonyl)anilino] 1, 3, 5-triazine).

According to the present invention, a low water-soluble substance having, in the chemical structure thereof, two or more 6-membered rings is employed. Examples of such a low water-soluble substance include UV-absorbing agents such as bisethylhexyloxyphenol methoxyphenyltriazine, and octyl triazone.
Of these, bisethylhexyloxyphenol methoxyphenyltriazine exhibits an excellent solubilization promoting effect when used in combination with compound (1).

No particular limitation is imposed on the amount of the low water-soluble substance contained in the water-containing composition of the present invention, as long as the low water-soluble substance can be maintained in a solubilized state in the composition at ambient temperature.
The relationship between the amount of the low water-soluble substance and that of compound (1) is as described above.
The specific amount of the low water-soluble substance may be determined in consideration, in addition to the above-described relationship, of the appropriate amount of the substance according to the nature thereof.

The amount of water contained in the water-containing composition of the present invention is the balance of the entire composition containing compound (1) and the low water-soluble substance, or the balance of the entire composition containing compound (1), the low water-soluble substance, and a common ingredient incorporated into the composition.

An embodiment of the water-containing composition of the present invention contains the aforementioned essential ingredients; i.e., water, a low water-soluble substance, and compound (1). By virtue of the presence of compound (1), the low water-soluble substance, which is originally difficult to dissolve in water, is readily solubilized. Preferably, the water-containing composition of the present invention is produced by, for example, dissolving a low water-soluble substance in compound (1) to thereby prepare a portion, and mixing the portion with water for solubilization of the low water-soluble substance.

The water-containing composition of the present invention containing only the aforementioned essential ingredients may be provided as an external-use composition used in the form of cosmetic composition or external-use agent. The external-use composition may be applied to the skin (including the scalp and hair) for providing one or more effects of the solubilized low water-soluble substance (e.g., medicinal effect and UV absorbing effect).

The water-containing composition of the present invention containing only the aforementioned essential ingredients may be employed as a base for preparing an external-use composition into which a common ingredient other than the essential ingredients is incorporated.
Specifically, a final external-use composition of interest may be produced by firstly producing the water-containing composition of the present invention, and secondly incorporating a common ingredient into the composition.

Such a common ingredient is selected in consideration of, for example, provision of medicinal effects, coloration, regulation of production parameters, adjustment of ionic strength, adjustment of pH, or stability of a drug product. Examples of the common ingredient include an excipient, a buffer, a salt, an antioxidant, a preservative, a perfume, a dye, a surfactant, a water-soluble vitamin, and a water-soluble drug. The common ingredient employed is generally a water-soluble substance. However, the common ingredient employed may be a low water-soluble substance, depending on the form of a final product. For example, the common ingredient may be a liquid oil ingredient having no 6-membered ring in the structure.

A final external-use composition of interest may be produced without using the above-described production process for an embodiment of the water-containing composition of the present invention containing only the essential ingredients. In such a case, the final external-use composition of interest may be produced through, for example, the following process: a water-soluble ingredient selected as a common ingredient is dissolved in water to thereby prepare an aqueous phase; and the aqueous phase is mixed with a portion prepared by dissolving a low water-soluble substance in compound (1), to thereby solubilize the low water-soluble substance. However, the method for producing the final external-use composition of interest is not limited to this production process, and may be appropriately selected or devised in consideration of the nature of an essential ingredient or selected common ingredient. As described above, the water-containing composition of the present invention may be provided in the form of water dispersion in which fine particles of a low water-soluble substance are dispersed in an aqueous phase.

### Examples

The present invention will next be described in more detail by way of examples, which should not be construed as limiting the invention thereto. Unless otherwise specified, the amount of an ingredient is represented by "mass%" with respect to the total amount of a composition into which the ingredient is incorporated.

### [Test Examples]

A low water-soluble substance was mixed with and dissolved in compound (1) or another surfactant through a customary method. The resultant portion was mixed with water, to thereby prepare a test sample shown in Tables 1 to 6 (Examples or Comparative Examples). The sample was evaluated according to the below-described criteria. The results of samples of each test system are shown in the corresponding table. In each table, blanks in rows corresponding to the amounts of ingredients incorporated represent "0 mass%."

### (1) Test immediately after preparation of test sample (a) Evaluation of dissolved state

The dissolved state of each test sample was visually evaluated according to the following criteria:

AA: transparent;
BB: generally transparent;
CC: turbid to semi-transparent; and
DD: phase separation observed.

### (b) Evaluation of transparency

The transparency of each test sample was evaluated through measurement of L value. L value was determined by means of a spectrophotometer (UV-160, product of Shimadzu Corporation). The transparency (L value) of each test sample was evaluated on the basis of the transparency of distilled water as a control (L value = 100) according to the following criteria:

90 or more: transparent;
70 to 90: generally transparent;
less than 70: turbid to semi-transparent; and
not determined: phase separation observed.

### (c) Sensation upon use

The sensation upon use of each test sample was evaluated by 10 expert panelists according to the following criteria:

0: 5 or more of the 10 expert panelists assessed that the test sample exhibited no stickiness;
Δ: 3 to 4 of the 10 expert panelists assessed that the test sample exhibited no stickiness; and
×: 0 to 2 of the 10 expert panelists assessed that the test sample exhibited no stickiness.

### (2) Test on change over time

Each of the above-prepared test samples was stored at 50°C for one month, and the L value of the thus-stored sample was measured in a manner similar to that described above. The stability over time of each test sample was evaluated on the basis of the difference between the L value determined immediately after preparation and the L value determined after one-month storage. The following criteria were used for evaluation. The L values determined after one-month storage are shown in the corresponding tables.

AA: difference in L value ≤ 2 (±);
BB: difference in L value ≤ 5 (±);
CC: difference in L value ≤ 10 (±); and
DD: difference in L value > 10 (±), or phase separation observed.

### <Test system 1>

**[Table 1]**

| | Comp. Ex. 1 | Comp. Ex. 2 | Comp. Ex. 3 | Ex. 1 | Ex. 2 | Ex. 3⁽¹⁾ | Ex. 4⁽¹⁾ |
|---|---|---|---|---|---|---|---|
| Ion-exchange water | to 100 | to 100 | to 100 | to 100 | to 100 | to 100 | to 100 |
| POE hydrogenated castor oil | 0.3 | - | - | - | - | - | - |
| POE-POP-added decyl tetradecyl ether | - | 0.3 | - | - | - | - | - |
| POE (30) phytosterol | - | - | 0.3 | - | - | - | - |
| POE (25)-poop (20) phytosterol | - | - | - | 0.3 | 1.5 | 0.4 | 1.0 |
| Bisethylhexyloxyphenol methoxyphenyltriazine | 0.1 | 0.1 | 0.1 | 0.1 | 0.1 | - | - |
| Finasteride | - | - | - | - | - | 0.1 | 0.1 |
| Dissolved state (immediately after preparation) | DD | DD | CC | AA | AA | AA | AA |
| L value (immediately after preparation) | 6 | 4 | 42 | 96 | 99 | 97 | 98 |

| | | | | | | | |
|---|---|---|---|---|---|---|---|
| (1) does not fall within the scope of the present invention | | | | | | | |

As shown in Table 1, the surfactant contained in the test sample of Comparative Example 1 or 2, which has no phytosteryl skeleton, exhibited poor ability to solubilize a low water-soluble substance; i.e., the surfactant was found to be unsuitable for solubilization of the substance.

Although the surfactant contained in the test sample of Comparative Example 3 has a phytosteryl skeleton, the surfactant does not have a polyoxypropylene skeleton. Therefore, the surfactant exhibited poor ability to solubilize a low water-soluble substance; i.e., the surfactant failed to solubilize the substance at a practical level.

Compound (1) contained in each of the test samples of Examples 1 to 4 solubilized a low water-soluble substance at a practically sufficient level.

### <Test system 2>

The test system shown in Table 2 focused on the ratio of the number of EO units (n) to that of PO units (m) in compound (1). Therefore, the ratio is also shown in the table.

**[Table 2]**

| | Ex. 5(*) | Ex. 6 | Ex. 7 | Ex. 8 | Ex. 9(*) | Ex. 10(*) |
|---|---|---|---|---|---|---|
| Ion-exchanqe water | to 100 | to 100 | to 100 | to 100 | to 100 | to 100 |
| POE (3)-POP (45) phytosterol | 0.5 | - | - | - | - | - |
| POE (15)-POP (30) phytosterol | - | 0.5 | - | - | - | - |
| POE (40)-POP (20) phytosterol | - | - | 0.5 | - | - | - |
| POE (20)-POP (5) phytosterol | - | - | - | 0.5 | - | - |
| POE (70)-POP (7) phytosterol | - | - | - | - | 0.5 | - |
| POE (100)-POP (2) phytosterol | - | - | - | - | - | 0.5 |
| 2,4,6-tuns[4-(2-ethylhexyloxycarbonyl)anilino] 1,3,5-triazine | 0.1 | 0.1 | 0.1 | 0.1 | 0.1 | 0.1 |
| EO/PO ratio of surfactant | 1/15 | 1/3 | 2/1 | 4/1 | 10/1 | 50/1 |
| Dissolved state (immediately after preparation) | AA | AA | AA | AA | AA | AA |
| L value (immediately after preparation) | 90 | 92 | 97 | 98 | 98 | 99 |
| Sensation upon use (immediately after preparation) | ○ | ○ | ○ | ○ | Δ | Δ |
| Stability over time | BB | AA | AA | AA | AA | AA |
| L value after 1-M storage at 50°C | 86 | 92 | 97 | 98 | 98 | 99 |

| | | | | | | |
|---|---|---|---|---|---|---|
| (*) does not fall within the scope of protection | | | | | | |

As shown in Table 2, the test sample of Example 5-in which the number (mol) of added oxyethylene units is 5 or less, and the ratio of the number (mol) of added oxyethylene units to that of added oxypropylene units is 1/50 or less-exhibited slightly poor stability over time.

The test sample of Example 10-in which the number (mol) of added oxypropylene units is 5 or less, and the ratio of the number (mol) of added oxyethylene units to that of added oxypropylene units is 50 or more-exhibited stickiness upon use.

The test example of Example 9, in which the ratio of the number (mol) of added oxyethylene units to that of added oxypropylene units is 5/1 or more, exhibited stickiness upon use.

The test samples of Examples 6 to 8-in which the number (mol) of added oxyethylene units is 5 to 100, the number (mol) of added oxypropylene units is 5 to 100, and the ratio of the number (mol) of added oxyethylene units to that of added oxypropylene units is 5/1 to 1/5-exhibited good appearance, sensation upon use, and stability over time.

### <Test system 3>

The test system shown in Table 3 focused on the ratio by mass of compound (1) to low water-soluble substance. Therefore, the ratio is also shown in the table.

**[Table 3]**

| | Ex.11⁽¹⁾ | Ex.12⁽¹⁾ | Ex. 13⁽¹⁾ | Ex. 14⁽¹⁾ |
|---|---|---|---|---|
| Ion-exchange water | to 100 | to 100 | to 100 | to 100 |
| POE (40)-POP (30) phytosterol | 3.0 | 1.5 | 0.8 | 0.4 |
| 4-tert-Butyl-4'-methoxydibenzoylmethane | 0.2 | 0.3 | 0.4 | 0.8 |
| Ratio of compound(1)/low water-soluble substance | 15/1 | 5/1 | 2/1 | 1/2 |
| Evaluation of appearance | AA | AA | AA | BB |
| L value | 98 | 99 | 96 | 85 |
| Sensation upon use | ○ | ○ | ○ | ○ |
| Stability over time | AA | AA | AA | BB |
| L value after 1-M storage at 50°C | 98 | 99 | 95 | 81 |

| | | | | |
|---|---|---|---|---|
| ⁽¹⁾ does not fall within the scope of the present invention | | | | |

The test sample of Example 14, in which the ratio by mass of compound (1) to low water-soluble substance is 1 or less, exhibited a slightly semi-transparent state.

The test samples of Examples 11 to 13, in which the ratio by mass of compound (1) to low water-soluble substance is 1 or more, exhibited good appearance, sensation upon use, and stability.

### <Test system 4>

**[Table 4]**

| | Ex.15⁽¹⁾ | Ex.16⁽¹⁾ | Ex.17⁽¹⁾ | Ex.18⁽¹⁾ | Ex.19⁽¹⁾ |
|---|---|---|---|---|---|
| Ion-exchanqe water | to 100 | to 100 | to 100 | to 100 | to 100 |
| POE (40)-POP (30) phytosterol | 0.4 | 0.4 | 0.4 | 0.4 | 0.4 |
| Vitamin E acetate | 0.2 | - | - | - | - |
| 2-Ethylhexyl p-methoxycinnamate | - | 0.2 | - | - | - |
| Hexyl diethylaminohydroxybenzoylbenzoate | - | - | 0.2 | - | - |
| 2-[2-Hydroxy-4-(2-ethylhexyl)phenoxy]-2H-benzotriazole | - | - | - | 0.2 | - |
| 2-Ethylhexyl-2-cyano-3,3-diphenyl acrylate | - | - | - | - | 0.2 |
| Evaluation of appearance | AA | AA | AA | AA | AA |
| L value | 95 | 97 | 94 | 98 | 96 |
| Stability over time | BB | BB | AA | AA | AA |
| L value after 1-M storage at 50°C | 91 | 93 | 94 | 97 | 96 |

| | | | | | |
|---|---|---|---|---|---|
| ⁽¹⁾ does not fall within the scope of the present invention | | | | | |

Each of the test samples of Examples 15 and 16, which contains a low water-soluble substance having only one 6-membered ring in the chemical structure thereof, exhibited good appearance (i.e., the substance was solubilized by compound (1)), but failed to achieve best results in terms of stability over time.

Each of the test samples of Examples 17 to 19, which contains compound (1) and alow water-soluble substance having two or more 6-membered rings in the chemical structure thereof, exhibited good appearance and stability.

### <Test system 5>

The test system shown in Table 5 employed a formulation (external-use agent) containing common ingredients in addition to essential ingredients of the water-containing composition of the present invention. For preparation of each test sample, the corresponding common ingredients (water-soluble ingredients) were mixed with ion-exchange water to thereby prepare an aqueous phase portion, and subsequently the aqueous phase portion was mixed with a portion prepared by dissolving a low water-soluble substance in compound (1).

**[Table 5]**

| | Ex. 20 | Ex. 21 | Ex. 22 | Ex. 23 |
|---|---|---|---|---|
| Ion-exchange water | to 100 | to 100 | to 100 | to 100 |
| Ethanol | 5.0 | 5.0 | 5.0 | 5.0 |
| Dynamite glycerin | 3.0 | 3.0 | 3.0 | 3.0 |
| Dipropylene glycol | 2.0 | 2.0 | 2.0 | 2.0 |
| POE (14)-POP (7) dimethyl ether | 1.0 | 1.0 | 1.0 | 1.0 |
| POE (40)-POP (30) phytosterol | 0.4 | 0.4 | 0.4 | 0.4 |
| Citric acid | 0.05 | 0.05 | 0.05 | 0.05 |
| Sodium citrate | 0.05 | 0.05 | 0.05 | 0.05 |
| Sodium ascorbate | 2.0 | - | - | - |
| Tranexamic acid | 1.0 | 2.0 | 0.5 | - |
| Potassium 4-methoxysalicylate | - | - | 1.0 | 1.0 |
| Phenoxyethanol | 0.5 | 0.5 | 0.5 | 0.5 |
| Perfume | 0.01 | 0.01 | 0.01 | 0.01 |
| Red No. 227 | 0.0001 | 0.0001 | 0.0001 | 0.0001 |
| Bisethylhexyloxyphenol methoxyphenyltriazine | 0.1 | 0.1 | 0.1 | 0.1 |
| Evaluation of appearance | AA | AA | AA | AA |
| L value | 97 | 98 | 96 | 99 |
| Sensation upon use | ○ | ○ | ○ | ○ |
| Stability over time | AA | AA | AA | AA |
| L value after 1-M storage at 50°C | 96 | 98 | 96 | 98 |

As is clear from data on the test samples of Examples 20 to 23, the original effects of the water-containing composition of the present invention are not impeded even in a system containing common ingredients.

<Test system 6>

The test system shown in Table 6 employed a formulation (hair cosmetic composition) containing common ingredients in addition to essential ingredients of the water-containing composition of the present invention. For preparation of each test sample, the corresponding common ingredients (water-soluble ingredients) were mixed with ion-exchange water to thereby prepare an aqueous phase portion, and subsequently the aqueous phase portion was mixed with a portion prepared by dissolving a low water-soluble substance in compound (1).

**[Table 6]**

| | Ex. 24⁽¹⁾ | Ex. 25 | Ex. 26 |
|---|---|---|---|
| Ion-exchange water | to 100 | to 100 | to 100 |
| Ethanol | 10 | 10 | 10 |
| Propylene glycol | 2.0 | 2.0 | 2.0 |
| Dipropylene glycol | 1.0 | 1.0 | 1.0 |
| POE (14)-POP (7) dimethyl ether | 1.0 | 1.0 | 1.0 |
| POE (20)-POP (10) phytosterol | 0.8 | 0.8 | 0.8 |
| Citric acid | 0.05 | 0.05 | 0.05 |
| Sodium citrate | 0.05 | 0.05 | 0.05 |
| Finasteride | 0.1 | 0.1 | - |
| Stearyltrimethylammonium chloride | 0.1 | 0.1 | 0.1 |
| Phenoxyethanol | 0.5 | 0.5 | 0.5 |
| Perfume | 0.01 | 0.01 | 0.01 |
| Bisethylhexyloxyphenol methoxyphenyltriazine | - | 0.1 | 0.1 |
| Evaluation of appearance | AA | AA | AA |
| L value | 99 | 99 | 97 |
| Sensation upon use | ○ | ○ | ○ |
| Stability over time | AA | AA | AA |
| L value after 1-M storage at 50°C | 99 | 98 | 97 |

| | | | |
|---|---|---|---|
| ⁽¹⁾ does not fall within the scope of the present invention | | | |

As is clear from data on the test samples of Examples 24 to 26, the original effects of the water-containing composition of the present invention are not impeded even in a system containing common ingredients.

## Claims

1. A water-containing composition comprising
water;
at least one polyoxyethylene-polyoxypropylene-added phytosterol or phytostanol represented by the following formula (1): 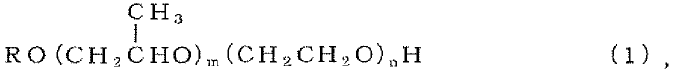 wherein R represents a phytosterol residue or a phytostanol residue, m is a number from 5 to 50, and n is a number from 10 to 50, the ratio of m to n being 1:5 to 5:1, the amount of the polyoxyethylene-polyoxypropylene-added phytosterol or phytostanol (1) being 10 mass% or less on the basis of the entirety of the composition; and
at least one low water-soluble substance which has, in the chemical structure thereof, two or more 6-membered rings, the low water-soluble substance being a triazine UV-absorbing agent,
wherein the low water-soluble substance is in a solubilization state.

2. The water-containing composition according to claim 1, which consists of water; the polyoxyethylene-polyoxypropylene-added phytosterol or phytostanol represented by the formula (1); and the low water-soluble substance.

3. The water-containing composition according to claim 1 or 2, wherein the ratio by mass of polyoxyethylene-polyoxypropylene-added phytosterol or phytostanol (1) to low water-soluble substance is 1 or more.

4. The water-containing composition according to any of claims 1 to 3, wherein the phytosterol residue is one or more phytosterol residues selected from the group consisting of a sitosterol residue, a campesterol residue, a stigmasterol residue, a brassicasterol residue, an avenasterol residue, and an ergosterol residue.

5. The water-containing composition according to any of claims 1 to 4, wherein the phytostanol residue is one or more phytostanol residues selected from the group consisting of a sitostanol residue, a campestanol residue, a stigmastanol residue, a brassicastanol residue, an avenastanol residue, and an ergostanol residue.

6. The water-containing composition according to any of claims 1 to 5, wherein the triazine UV-absorbing agent is bisethylhexyloxyphenol methoxyphenyltriazine or 2,4,6-tris[4-(2-ethylhexyloxycarbonyl)anilino] 1,3,5-triazine.

7. The water-containing composition according to any of claims 1 to 6, which is an external-use composition.

8. The water-containing composition according to claim 7, wherein the external-use composition is a cosmetic composition.

9. The water-containing composition according to any of claims 1 to 6, which is a base for producing an external-use composition.

## Patentansprüche

1. Wasser enthaltende Zusammensetzung, umfassend
Wasser;
mindestens ein Polyoxyethylen-Polyoxypropylen-addiertes Phytosterol oder Phytostanol, das durch die folgende Formel dargestellt wird (1): 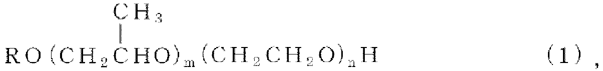 wobei R für einen Phytosterolrest oder einen Phytostanolrest steht, m eine Zahl von 5 bis 50 ist und n eine Zahl von 10 bis 50 ist, das Verhältnis von m zu n 1:5 bis 5:1 beträgt, die Menge des Polyoxyethylen-Polyoxypropylen-addierten Phytosterols oder Phytostanols (1) 10 Massenprozent oder weniger, bezogen auf die Gesamtheit der Zusammensetzung, beträgt; und
mindestens eine gering wasserlösliche Substanz, die in ihrer chemischen Struktur zwei oder mehr 6-gliedrige Ringe aufweist, wobei die gering wasserlösliche Substanz ein Triazin-UV-Absorptionsmittel ist,
wobei die gering wasserlösliche Substanz in einem Solubilisierungszustand vorliegt.

2. Wasser enthaltende Zusammensetzung nach Anspruch 1, die aus Wasser, Polyoxyethylen-Polyoxypropylen-addiertem Phytosterol oder Phytostanol der Formel (1) und der gering wasserlöslichen Substanz besteht.

3. Wasser enthaltende Zusammensetzung nach Anspruch 1 oder Anspruch 2, wobei das Massenverhältnis des Polyoxyethylen-Polyoxypropylen-addierten Phytosterols oder Phytostanols (1) zu der gering wasserlöslichen Substanz 1 oder mehr beträgt.

4. Wasser enthaltende Zusammensetzung nach einem der Ansprüche 1 bis 3, wobei der Phytosterolrest ein oder mehrere Phytosterolrest(e), die aus der Gruppe ausgewählt sind, die aus einem Sitosterolrest, einem Campesterolrest, einem Stigmasterolrest, einem Brassicasterolrest, einem Avenasterolrest und einem Ergosterolrest besteht, ist.

5. Wasser enthaltende Zusammensetzung nach einem der Ansprüche 1 bis 4, wobei der Phytostanolrest ein oder mehrere Phytostanolrest(e), die aus der Gruppe ausgewählt sind, die aus einem Sitostanolrest, einem Campestanolrest, einem Stigmastanolrest, einem Brassicastanolrest, einem Avenastanolrest und einem Ergostanolrest besteht, ist.

6. Wasser enthaltende Zusammensetzung nach einem der Ansprüche 1 bis 5, wobei das Triazin-UV-Absorptionsmittel Bisethylhexyloxyphenol-methoxyphenyltriain oder 2,4,6-Tris[4-(z-ethylhexyloxycarbonyl)anilino]1,3,5-triazin ist.

7. Wasser enthaltende Zusammensetzung nach einem der Ansprüche 1 bis 6, die eine Zusammensetzung zur externen Verwendung ist.

8. Wasser enthaltende Zusammensetzung nach Anspruch 7, wobei die Zusammensetzung zur externen Verwendung eine kosmetische Zusammensetzung ist.

9. Wasser enthaltende Zusammensetzung nach einem der Ansprüche 1 bis 6, die eine Basis zur Herstellung einer Zusammensetzung zur externen Verwendung ist.

## Revendications

1. Composition aqueuse comprenant
de l'eau ;
au moins un phytostérol ou phytostanol possédant du polyoxyéthylène-polyoxypropylène ajouté représenté par la formule générale (1) : 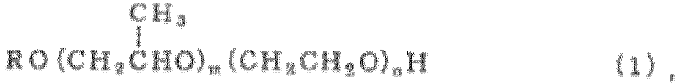 dans laquelle R représente un résidu phytostérol ou un résidu phytostanol, m est un nombre entier compris entre 5 et 50, et n est un nombre entier compris entre 10 et 50, le rapport de m à n étant de 1:5 à 5:1, la quantité du phytostérol ou phytostanol possédant du polyoxyéthylène-polyoxypropylène ajouté (1) étant de 10 % en masse ou moins sur la base de la totalité de la composition ; et
au moins une substance faiblement soluble dans l'eau qui a, dans la structure chimique de celle-ci, au moins deux cycles à 6 chaînons, la substance faiblement soluble dans l'eau étant un agent absorbant les UV de type triazine,
dans laquelle la substance faiblement soluble dans l'eau est dans un état de solubilisation.

2. Composition aqueuse selon la revendication 1, qui comprend de l'eau ; le phytostérol ou phytostanol possédant du polyoxyéthylène-polyoxypropylène ajouté représenté par la formule (1) ; et la substance faiblement soluble dans l'eau.

3. Composition aqueuse selon la revendication 1 ou 2, dans laquelle le rapport en masse du phytostérol ou phytostanol possédant du polyoxyéthylène-polyoxypropylène ajouté (1) à la substance faiblement soluble dans l'eau est de 1 ou plus.

4. Composition aqueuse selon l'une quelconque des revendications 1 à 3, dans laquelle le résidu phytostérol est un ou plusieurs résidu(s) phytostérol choisi(s) dans le groupe constitué par un résidu sitostérol, un résidu campestérol, un résidu stigmastérol, un résidu brassicastérol, un résidu avenastérol et un résidu ergostérol.

5. Composition aqueuse selon l'une quelconque des revendications 1 à 4, dans laquelle le résidu phytostanol est un ou plusieurs résidu(s) phytostanol choisi(s) dans le groupe constitué par un résidu sitostanol, un résidu campestanol, un résidu stigmastanol, un résidu brassicastanol, un résidu avenastanol et un résidu ergostanol.

6. Composition aqueuse selon l'une quelconque des revendications 1 à 5, dans laquelle l'agent absorbant les UV de type triazine est le biséthylhexyloxyphénol-méthoxyphényltriazine ou le 2,4,6-tris[4-(2-éthylhexyloxycarbonyl)anilino]1,3,5-triazine.

7. Composition aqueuse selon l'une quelconque des revendications 1 à 6, qui est une composition à usage externe.

8. Composition aqueuse selon la revendication 7, laquelle composition à usage externe est une composition cosmétique.

9. Composition aqueuse selon l'une quelconque des revendications 1 à 6, qui est une base pour produire une composition à usage externe.
